# EUROPEAN PATENT APPLICATION

(11) **EP 2 705 814 A1**
(43) Date of publication of application: **12.03.2014**
(21) Application number: 12183517.7
(22) Date of filing: 07.09.2012
(51) Int. Cl.: A61F 15/00

(54) **Device for applying a bandage around a limb**

(71) Applicant: Dona +, 5504 KA Veldhoven (NL); P&H Adviseurs Bouw-en Vastgoed B.V., 5504 KA Veldhoven (NL)
(72) Inventor: Dieleman, Maria, 6417 GR Heerlen (NL); van Rens, Piet Christiaan Jozef, 5721 LE Asten (NL); Dona, Marinus Josephus Jacobus, 5504 KA Veldhoven (NL); Peeters, Eugène Adrianus Franciscus Maria, 5504 KA Veldhoven (NL)
(74) Representative: Nederlandsch Octrooibureau

(57) **Abstract**

A device (1) for use in a process of applying a bandage around a limb comprises a ring (4) which is adapted to surround the limb (2, 3); a guide (5) for guiding the ring (4), wherein the ring (4) is movably connected to the guide (5); a rotatable support for supporting a bandage roll (10), which support is located in the ring (4); a motor for driving the support; a drive circuit (40) for driving the motor; an interface (50, 51) for receiving input information representing a desired tension of the bandage during a bandage applying process; and a controller (41) for controlling operation of the motor during a bandage applying process through the drive circuit (40) by setting an output torque of the motor.

## Description

The present invention relates to a device for applying a bandage around a limb, comprising a ring which is adapted to surround the limb; a guide for guiding the ring, wherein the ring is movably connected to the guide; and a rotatable support for supporting a bandage roll, which support is located in the ring.

For various medical reasons, it may be necessary to apply a bandage around a limb of a person or an animal in order to exert a certain pressure on the limb. For example, when a person is suffering from fluid-filled areas in the leg, a doctor may determine that the swollen leg is subjected to compression bandaging every day or every other day, so that it is eventually possible to use an elastic support stocking on the leg and to further treat the leg by means of the stocking. Applying a bandage for the purpose of realizing a prescribed pressure on a limb is a labour-intensive and heavy task for medical staff members, wherein they are at risk of getting back pain or other physical problems. Furthermore, it is very difficult for medical staff members to perform the process of applying a bandage in such a way that a predetermined pressure is actually realized, as it is only possible for them to rely on feeling based on experience. As a result, in many practical cases, it appears that the pressure is too high or too low. Hence, there is a need for a device for applying a bandage around a limb, which is adapted to automatically unwind a bandage roll and wrap the bandage around a limb while obtaining a correct pressure on the limb, and which may be operated by a patient without intervention of a medical staff member.

NL 1037955 discloses a device which is capable of performing a process of applying a bandage around a limb, especially a leg, starting from the toes and finishing at a desired position on the leg. To this end, the device has a ring which is adapted to surround the foot or the leg and to be displaced in a direction which more or less equals a longitudinal direction of the foot and the leg, wherein the ring is arranged on a guide in such a way as to be movable along the guide and to be tiltable with respect to the guide. Furthermore, the ring carries a rotatable support for supporting a bandage roll. The ring may comprise a fixedly arranged basic ring and a rotatable ring portion, wherein the support is located at the rotatable ring portion. Alternatively, the support may be displaceable in the ring by means of a suitable element which is connected to the support on the one hand and which is movable with respect to the ring on the other hand.

In order to make a start with a bandage applying process, it is possible to use a foot support, which may be a stick, for example. After the bandage applying process, the patient can allow his/her foot to slide from the stick, wherein the stick is retracted from the bandage.

The bandage is applied starting at the foot and moving to a certain position on the leg. In a first instance, the ring of the device is in a position for surrounding the foot. As soon as the heel is reached, the ring tilts in order to assume an orientation which is appropriate for following the leg. The positioning of the guide is adapted to the desired movement of the ring. A connection element which is present between the guide and the ring is displaceable, moving in a downward direction along the guide during the process of tilting about the heel, and subsequently moving in an upward direction along the guide again in following the leg.

Besides the device as described in the foregoing, various types of hand-held bandage applicators are known in the art. For example, WO 2011/025813 discloses a bandage applicator comprising a handle to be held by a user of the applicator, wherein the handle is coupled to a support in the form of a spindle. Furthermore, the applicator comprises a brake mechanism which is integral with the handle for controlling a tension of the bandage as it is applied to a limb. The bandage may be pre-wound on a spool that rotates around the spindle. Actuation of the braking mechanism increases pressure on the rotating spool in order to increase tension on the bandage. Alternatively, the spindle rotates in conjunction with a shaft within the handle. In that case, the braking mechanism applies pressure against the rotating shaft to increase tension in the bandage. Pre-wound cartridges of rolled bandage material may be successively loaded to the applicator's spindle for repeated usage of the applicator.

US 5,065,865 discloses a holder for a bandage and a tension roller journalled in the holder. The roller is controlled so that the force required to roll it is adjustable. The bandage is entrained over the roller and the surface of the roller is formed so that there is positive engagement of the bandage with the roller whereby the tension in the bandage is controlled. The holder includes an enclosure formed by end walls and curved piece, which serves to hold a bandage roll. From the enclosure, the bandage runs under an extension, thence over a drum or roller having an axle journalled in the side walls in front of the enclosure. This axle may take the form of a long bolt having a head at one end and a thumb nut threaded onto the bolt at the other. Bushings on the axle and between the ends of the roller and the side walls of the enclosure hold the rollers properly spaced, and provide braking for the roller as the thumb nut is tightened to press the side walls against the bushings. In order to have a possibility of replicating the pressure, a scale may be provided on the side wall under the nut. By noting the position of the nut on the scale when proper pressure is applied, and then using a similar setting of the nut, a duplicate -or near duplicate- of the pressure should be achieved.

GB 2 333 506 discloses a bandage applicator which comprises a support in the form of an axle around which a bandage having an adhesive element is wound. The axle is rotated within a handle, and a tension regulator is provided to control the degree of friction between the handle and the axle as the bandage is unwound from the applicator.

GB 2 369 815 discloses a bandage applicator which comprises a cylindrical body rotatable within a housing which forms a handle, and means at one end of the cylindrical body for mounting a bandage roll. The housing may be provided by two opposing half-casings, which are preferably cylindrical and concentric with the cylindrical body. The half-casings attach around a projection located on the cylindrical body, and further comprise interlocking studs and opposing grooves. The cylindrical body may also have a cylindrical extension fitted with longitudinal slots for engaging longitudinal ridges on a tubular body. The cylindrical body may have a projection in a region adjacent the handle, which, when pressurized by a user's finger, brakes the rotation of the cylindrical body within the housing in order to regulate the tension of the bandaging. The projection may further comprise linear ridges to facilitate the grip of the user's finger. The bandage material may be rolled around the tubular body, attached via an adhesive element. The bandage material may also have an adhesive element to allow the beginning of the bandage to be found.

It appears from the description of the various documents mentioned in the foregoing that it is known in the art to have a bandage applicator in which regulating the tension of the bandage involves applying pressure in order to have a braking action directly on the bandage, or on the support on which a bandage roll is mounted.

It is an objective of the present invention to provide a device for applying a bandage around a limb, in which the tension prevailing inside a bandage to be applied to a limb is set according to another principle than the known principle of performing a braking action in a mechanical manner, i.e. by having friction between at least two elements which are moving with respect to each other.

The objective of the present invention is achieved by means of a device for applying a bandage around a limb, comprising a ring which is adapted to surround the limb; a guide for guiding the ring, wherein the ring is movably connected to the guide; a rotatable support for supporting a bandage roll, which support is located in the ring; a motor for driving the support; a drive circuit for driving the motor; an interface for receiving input information representing a desired tension of the bandage during a bandage applying process; and a controller for controlling operation of the motor during a bandage applying process through the drive circuit, which is connected to the interface for receiving the input information from the interface, which is configured to determine a value representing an output torque of the motor on the basis of the input information, and which is connected to the drive circuit for setting the value in the drive circuit.

According to the present invention, controlling the tension in a bandage in a process of wrapping the bandage around a limb involves controlling an output torque of the motor which is connected to the support. Hence, realizing a correct tension is done in a most accurate way by setting an output torque instead of performing a braking action as known in the art. During operation of the device according to the present invention, the motor acts to retract the bandage as long as a pulling force is exerted on the bandage in a process of wrapping the bandage around a limb by means of the support of the bandage roll performing a movement around the limb in the ring. In case the movement of the support with respect to the limb is stopped, the bandage tends to wind around the support again, as a result of which sagging of the bandage and loss of tension in the bandage are prevented. By controlling the output torque of the motor, it is guaranteed that the correct tension is always prevailing in the bandage, independent of the speed at which the support moves around the limb.

The device according to the present invention is very easy to use, wherein a bandage applying process is performed automatically, so that medical staff members are relieved from a labour-intensive, heavy and difficult task, and wherein a prescribed pressure of the bandage is accurately realized. As explained in the foregoing, the exact tension in the bandage is obtained automatically by appropriate control of the motor, particularly the output torque of the motor, which results in a certain pulling force through the bandage roll.

The exact value of the output torque is determined on the basis of input information. When the input information represents a higher tension of the bandage, the output torque is higher as well. The input information can be varied over time during a bandage applying process if so desired, for example, depending on the position of the bandage on a limb, wherein the output torque of the motor is varied in a similar manner.

In a practical embodiment of the device according to the present invention, the motor is an electromotor, wherein the controller is configured to determine a value representing a supply of current to the motor on the basis of the input information, and to control the drive circuit for realizing the value. Hence, the working principle of this embodiment is based on the fact that the output torque of an electromotor is determined by the current that is supplied to the motor, wherein the current may be set. In this embodiment, the controller may be configured to determine a voltage to be set in the drive circuit. Setting a current on the basis of an input voltage is possible when the drive circuit is adapted to realize a direct relation between the voltage and the current. For example, this is the case when the drive circuit comprises a metal-oxide-semiconductor field-effect transistor (MOSFET), an operational amplifier and a resistor, wherein the operational amplifier is connected to the controller for receiving an incoming voltage set by the controller, and wherein the operational amplifier is arranged for letting a voltage across the resistor be the same as the incoming voltage and thereby determining a value of a current in the drive circuit and determining an opened/closed state of the MOSFET.

For realizing a predetermined tension in a bandage, the output torque of the motor is set at an appropriate value by setting the current to be supplied to the motor. As long as the current is kept at a constant value, the output torque is at a constant value, and the tension is at a constant value (not taking into account a small variation on the basis of the fact that the diameter of the bandage roll decreases during unwinding). By using a circuit as mentioned, in which a MOSFET, an operational amplifier and a resistor are arranged besides the motor and a power source, it is possible to link the value of the current directly to a value of an input voltage. The MOSFET is a type of transistor, which can be used as an electrically controlled switch. In this case, the operational amplifier is used for determining the opened/closed state of the MOSFET. The operational amplifier is an electrical component which can be used as a voltage follower, wherein a voltage drop across the resistor equals the input voltage at the operational amplifier. In this way, the input voltage can be used to directly determine the current to be supplied to the motor.

The interface which is used for transferring input information to the controller of the device according to the present invention can comprise a potentiometer, wherein a user can set the correct tension for a bandage by simply rotating a knob, for example.

The device according to the present invention offers a possibility of receiving input information in an automated manner. In particular, the interface may be adapted to communicate with a computer through the Internet. Additionally or alternatively, the interface may be adapted to read information from a digital card.

On the other hand, it is possible for the device to provide information about bandage applying processes which have been performed by means of the device. To that end, the device may comprise a memory for storing information regarding the voltage in the drive circuit for driving the motor, which can be used as a representative of the tension in the bandage as explained in the foregoing. In general, the memory may be adapted to store information regarding the value representing the output torque of he motor as set in the drive circuit. In a practical embodiment, the device further comprises a transmitter for transmitting information from the memory to a location outside of the device. In order to keep the number of wires which are used with the device to a minimum, the transmitter may be adapted to transmit information in a wireless fashion.

According to another option, the device may be equipped with an encoder for registering length and position of applied bandages, wherein the encoder is located at an output shaft of the motor. Also, it is possible to equip the device with a measuring device which is adapted to measuring dimensions of limbs, particularly a thickness of limbs. By performing measurements of the thickness of limbs, it is possible to check whether improvements are made by using the bandages and applying a certain pressure to the limbs by means of the bandages, and to which extent the thickness is decreased.

All in all, various ways of providing information to the device and obtaining information from the device are possible within the framework of the present invention. When the Internet is used, for example, it is possible for a doctor to monitor the bandage applying processes which are performed on a patient, and also to provide appropriate information for future processes. Especially when the electromotor as described in the foregoing and the electrical circuit for controlling the current to be supplied to the motor are used, it is relatively simple to equip the device with suitable components for realizing exchange of information between the device and the external world. If such components are used, the device may even play a role in avoiding situations in which a patient is left unattended while something is seriously wrong. When a patient is supposed to use the device himself/herself on a regular basis, and it happens that information is not updated at a prescribed time, it may be concluded that something is wrong with the patient, so that appropriate action may be taken and the patient may be visited for checking if there are problems, indeed.

In a further automated embodiment, the device according to the present invention may comprise a scanner for reading information on a bandage roll representing the type of the bandage roll, wherein the scanner is connected to the controller. Various types of bandage rolls may be provided with various barcodes, and the scanner may be a barcode reader. It may be relevant to have information about the type of bandage roll, especially in view of the fact that rolls of self-adhesive bandage exist, which require a higher torque for unwinding.

The above-described and other aspects of the present invention will be apparent from and elucidated with reference to the following detailed description of a device for automatically applying a bandage around a limb, which is adapted to set a required tension in a bandage during a bandage applying process by controlling an output torque of a motor which is present in the device for driving a support for supporting a bandage roll.

The present invention will now be explained in greater detail with reference to the figures, in which equal or similar parts are indicated by the same reference signs, and in which:
figure 1 diagrammatically shows a side view of the device according to the present invention and a person's lower leg and foot;
figures 2a, 2b and 2c illustrate a movement which is performed during a bandage applying process by a ring of the device;
figure 3 shows a sectional view of an assembly for supporting and driving a bandage roll in the device; and
figure 4 is an electrical diagram of a controller of the device and a drive circuit for driving a motor which is present in the device.

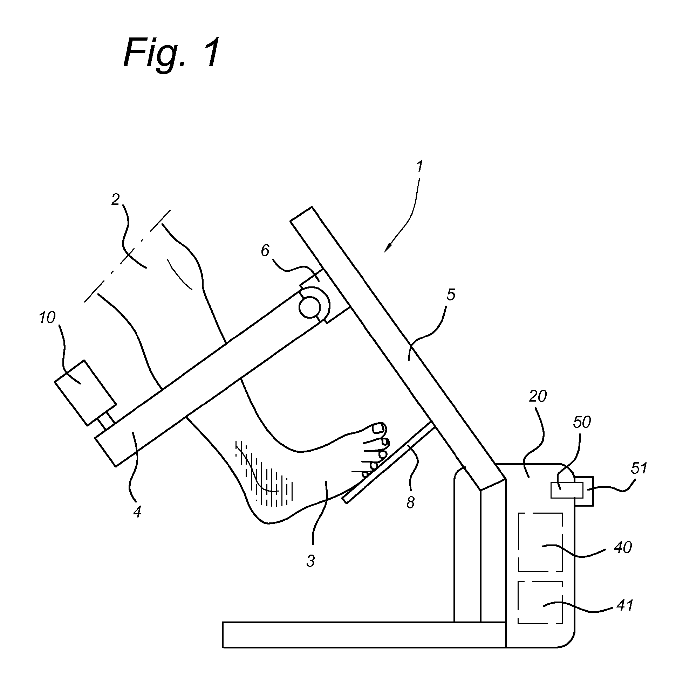

A device 1 which is designed to be used for applying a bandage to a person's leg 2 and foot 3, and which will hereinafter be referred to as bandage applying device 1, comprises the following components, as shown in figure 1:
- a ring 4 for surrounding the leg 2 and foot 3;
- a guide 5 and a connection element 6 connecting the ring 4 to the guide 5, wherein, in the shown example, the guide 5 has a somewhat inclined orientation with respect to the vertical, in a normal position of the device 1, i.e. an operational position as shown in figure 1;
- a support for supporting a bandage roll 10, which support is located in the ring 4;
- a foot support 7 for supporting the leg 2 and foot 3, and for keeping the foot 3 in an appropriate position during a bandage applying process; and
- an electrical box 20 for accommodating electrical components as will be explained later on.

For sake of completeness, it is noted that a bandage is generally known. Normally, the bandage has a length of several meters, and is available in a condition in which it has been wound to form a roll 10.

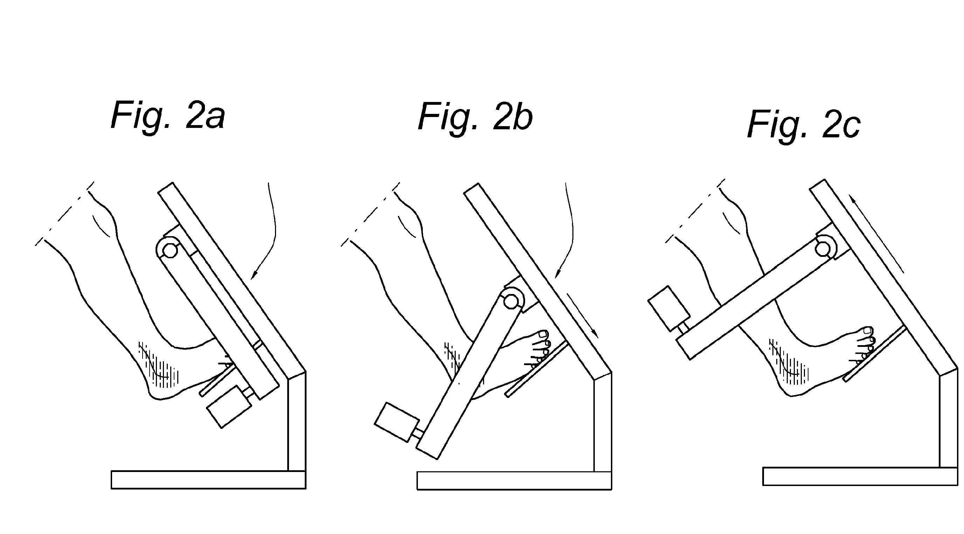

Figures 2a, 2b and 2c illustrate the functioning of the bandage applying device 1, wherein, for sake of clarity, the electrical box 20 of the device 1 is not shown. In general, the bandage applying device 1 operates to move the ring 4 with a bandage roll 10 as supported on the support of the device 1 from the toes to a position on the leg 2, along the foot 3 and the leg 2, wherein a rotation of the support and the bandage roll 10 supported thereby around the foot 3 and the leg 2 is realized, following an imaginary circle defined by the ring 4.

A starting position of the bandage applying device 1 is shown in figure 2a. In this position, the ring 4 is orientated such as to enable a process of enwrapping the foot 3. In the shown example, this is an orientation in which the ring 4 extends substantially parallel to the guide 5. Starting from this orientation, the ring 4 tilts with respect to the guide 5, as indicated by means of a curved arrow in figure 2a, while the ring 4 is moved further and further in the direction of the heel. In the process, the connection element 6 moves in a downward direction along the guide 5, as indicated by means of a straight arrow in figure 2b.

When the ring 4 is at the location of the heel, the ring 4 may start following the leg 2, in an upward direction. The ring 4 is tilted to an increasing extent until the ring 4 extends substantially perpendicular with respect to the guide 5. The connection element 6 moves in an upward direction along the guide 5, as indicated by a straight arrow in figure 2c.

During the entire bandage applying process, the support and the bandage roll 10 supported thereby are moved around the foot 3 and the leg 2, so that the bandage is wrapped around the foot 3 and the leg 2. In order to fixate an end section of the bandage at the start, the bandage applying device 1 can be provided with any suitable element. The end section of the bandage can be provided with a loop, which is made to engage with the element in such a case.

At the other end section, the bandage is fixated as well, for example, by means of tape which is wrapped along with the bandage. When the bandage is placed on the support, the tape is arranged around the support first, which is wrapped around the support with the sticky side outward, after which the rest of the bandage is put in place. In such a case, when the bandage is applied to the foot 3 and the leg 2, and the end of the bandage is reached, the tape follows automatically, which ends up on the bandage with its sticky side facing the bandage and, in doing so, attaches the end of the bandage to another portion of the bandage.

In the following, a number of remarks and considerations are given in respect of the bandage applying device 1 according to the present invention.

The bandage applying device 1 is capable of realizing an enwrapping process of the foot 3 (starting at the smallest toe) to the knee, in a totally or partially autonomous manner, wherein the pressure exerted by the bandage on the limb 2, 3 is accurately controlled. The support for supporting the bandage roll 10 can perform the process independently, and is particularly capable of performing an appropriate movement with respect to the foot 3 and the leg 2. The bandage can be a standardized bandage, having a loop at one end section and tape at another end section. A programme can be used for controlling the movement of the support, which programme can be a self-leaming programme if so desired.

The bandage applying device 1 can be used for applying a bandage to a foot 3 and a lower leg. It is also possible that the bandage applying device 1 is suitable for applying a bandage to other body parts, particularly only a foot 3 from the smallest toe to the ankle, only a lower leg, an entire leg 2, or an arm.

The beneficial role of the bandage applying device 1 according to the present invention in society has a number of aspects. In the first place, less back-related problems of medical staff members are to be expected. In the second place, it is possible to apply the bandage with a reproducible/predictable pressure. In the third place, independency of the patient is increased, whereby a lack of medical staff members can be compensated for. This is all the more important in view of the fact that an increase of the number of patients who need to make use of compression bandaging is expected in the nearby future. All in all, the present invention provides for a well-controlled process and a correct handling of a diagnosis-based advice.

In respect of a bandage applying device 1 which is suitable for enwrapping both a foot 3 and a portion of a leg 2, as is the case in the example disclosed on the basis of figures 1, 2a, 2b and 2c, the following considerations are true:
- The foot 3 needs to be enwrapped at approximately 90° with respect to the lower leg.
- The bandage applying process starts at the foot 3 (coupling to toe).
- The bandage is mechanically wrapped around the foot 3 and the leg 2 in a helix pattern. In the process, usual prescriptions are followed.
- The bandage is applied while uncoiling from the support, wherein the path which is followed utilizes the degrees of freedom in order to freely rotate around the foot 3 and the leg 2.
- During the bandage applying process, the pressure of the bandage on the limb 2, 3 is continuously controlled in order to accurately realize a prescribed pressure. - It is possible to place additional pieces in the bandage, which are fixed in their place as the bandage is applied, such as cotton wool for equally spreading the pressure, sensors, stockings, a medicinal patch, a drain, a prosthesis, etc. etc. For example, the patient can wear measuring stockings during a first treatment for measuring spreading of the pressure, so that it is possible to realize adjustment or corrections in the bandage applying process if it appears to be necessary to do so.
- The bandage applying device 1 can be equipped with programmable software which can be adjusted to a patient's diagnosis.
- The bandage applying device 1 can be equipped with means for determining dimensions of a limb 2, 3, especially a periphery of a leg 2. Such means can be located in the ring 4, so that it is possible for such means to move around the limb 2, 3 and to scan the limb 2, 3. In this way, results of the treatment of the limb 2, 3 through compression bandaging can be determined. Alternatively, it is possible to derive information about dimensions of a limb 2, 3, especially the periphery of the limb 2, 3, by registering a length of a bandage which has been used, possibly in relation to the number of turns made by means of the bandage.

In respect of the foot support 7, it is noted that this is primarily a component which supports the foot 3 during the bandage applying process, to a certain extent, for example, only at the location of the toes, or also at another location, possible only during a limited period of the process. By means of the foot support 7, it is possible for the foot 3 to stay positioned during the bandage applying process. Furthermore, the foot support 7 prevents the leg 2 from falling to the ground when being taken out of the bandage applying device 1. The foot support 7 can be a general component, but may also be especially adapted to physical features of the patient, particularly the shape of the foot 3. In order to keep the foot 3 in place, various means can be used if so desired, like a kind of shoe made of cloth, or an inflatable element for positioning a toe or the entire foot 3.

The bandage which is used can be a double woven bandage, can be provided with openings, can have different elastic properties along its length, can contain sensors, and/or can be provided with additional bands. The bandage may vary in properties like width, thickness and texture.

It is possible to have a displaceable platform for providing additional support to the foot 3 and the leg 2 as soon as the ring 4 of the bandage applying device 1 has moved beyond the ankle, by moving to a higher level at that instance.

The support for supporting a bandage roll 10 can be shaped in any suitable manner for allowing the bandage roll 10 to uncoil during the bandage applying process.

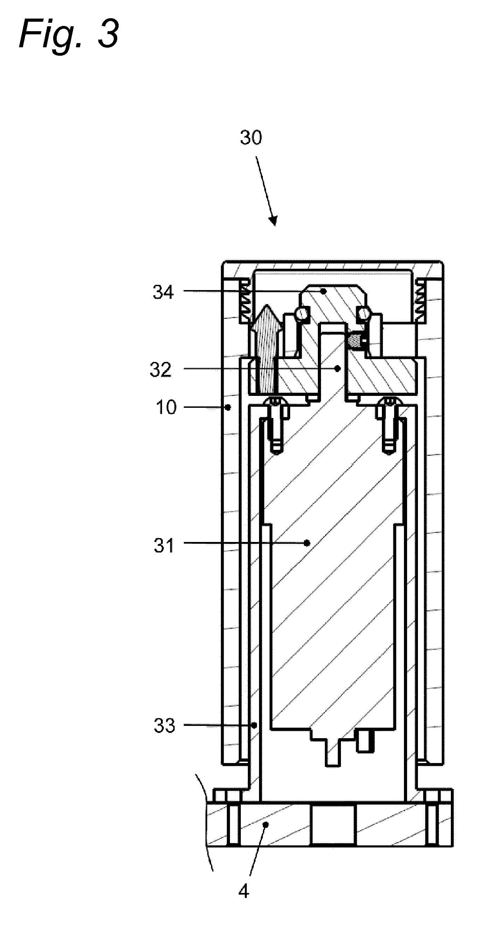

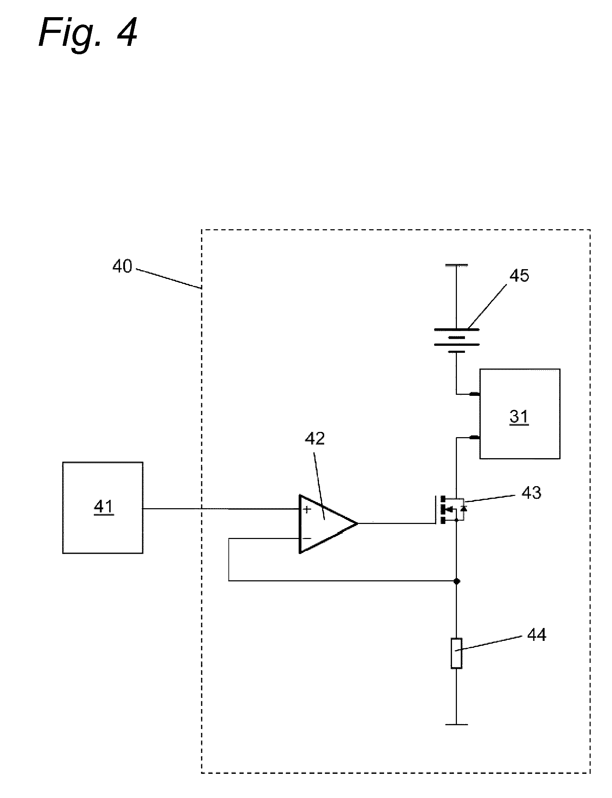

As mentioned in the foregoing, it is important to realize a prescribed pressure in a bandage as applied on a limb 2, 3 as accurate as possible, and the bandage applying device 1 according to the present invention is adapted to do just that. In figure 1, an electrical box 20 for accommodating electrical components which have a function in this respect is shown. Features of these components and other characteristics of the bandage applying device 1 which play a role in controlling the pressure in a bandage as applied on a limb 2, 3 will now be explained on the basis of figures 3 and 4.

Figure 3 shows a sectional view of an assembly 30 for supporting and driving a bandage roll 10, which is located in the ring 4 (of which only a portion is shown in figure 3), and illustrates the fact that the assembly 30 comprises an electromotor 31 having an output shaft 32, which is arranged in a housing 33, and further comprises a support 34 as mentioned earlier for supporting a bandage roll 10, wherein the support 34 is arranged on the output shaft 32 of the motor 31. Hence, the support 34 is driven by the motor 31 during operation of the bandage applying device 1. When it is intended to use the bandage applying device 1 for applying a bandage around a limb 2, 3, a bandage roll 10 is provided and coupled to the support 34. The support 34 can be equipped with any suitable means for establishing a coupling which is both releasable and reliable.

The capability of the bandage applying device 1 to realize a correct tension in the bandage during the bandage applying process is based on an accurate control of the operation of the motor 31, as will be explained in the following.

A principle underlying the design of the bandage applying device 1, particularly the design of the assembly 30 for supporting and driving a bandage roll 10, is that the tension prevailing in a bandage is not controlled by braking the bandage, but by using the motor 31 for realizing a certain torque at the support 34. By using a motor 31 and controlling the output torque of the motor 31, the tension in the bandage is accurately set, independent of the speed at which the bandage roll 10 is moved around a limb 2, 3 and exerts a pulling force at the bandage during such a movement. In fact, the movement of the bandage roll 10 causes the bandage to unwind from the roll 10 without influencing the tension prevailing in the bandage. If, during a bandage applying process, the bandage roll 10 is rotated at a higher speed around a limb 2, 3, the speed at which the bandage is unwound from the roll 10 is increased, while the tension in the bandage is not changed. If, during a bandage process, the movement of the bandage roll 10 is stopped, a force is exerted on the bandage in such a way that the bandage is inclined to be wound back on the roll 10 again until the movement is continued.

In general, for the purpose of controlling the operation of the motor 30 so that a desired tension is realized in a bandage, the bandage applying device 1 comprises a drive circuit for driving the motor 31, an interface for receiving input information representing the desired tension, and a controller which is connected to both the interface and the drive circuit. In the shown example, the drive circuit is a circuit 40 comprising electrical components as diagrammatically shown in figure 4, which are located inside the electrical box 20. The controller 41 can be a programmable chip, for example, and may be located inside the electrical box 20 as well. In figure 1, both the controller 41 and the drive circuit 40 are indicated by means of dashed boxes. In figure 4, the controller 41 is diagrammatically depicted in the form of a box which is connected to the drive circuit 40. Furthermore, in the shown example, the interface is in the form of a potentiometer 50 and an associated knob 51 by means of which a user of the bandage applying device 1 can adjust a setting of the potentiometer 50. For sake of completeness, it is noted that the bandage applying device 1 may be equipped with suitable wiring for connecting the potentiometer 50 to the controller 41.

The controller 41 is adapted to provide an input value to the drive circuit 40, particularly an input voltage which is determined by the controller 41 in relation to the setting of the potentiometer 50. The drive circuit 40 is designed to set an input current for the motor 31 on the basis of the input voltage, and to thereby determine an output torque of the motor 31 and an associated tension of the bandage. The input voltage is received by the drive circuit 40 at a side where a operational amplifier 42 is present. The drive circuit 40 further comprises a MOSFET 43, a resistor 44, and a power source 45 which may be the mains or a battery, for example. In this arrangement, the value of the current at which the motor 31 is operated is determined by the operational amplifier 42, the MOSFET 43 and the resistor 44. In particular, the MOSFET 43 is put in an opened state when the value of the current is too low, and the MOSFET 43 is put in a closed state when the value of the current is too high, wherein the operational amplifier 42 is applied for controlling the state of the MOSFET 43. The operational amplifier 42 is arranged in the circuit 40 in such a way as to be capable of serving as a voltage follower, wherein a voltage drop across the resistor 44 is always equal to the input voltage. On the basis of the well-known fact that the current is directly related to the voltage through a factor which is determined by the resistance of the resistor 44 (V = I * R, wherein V represents the voltage, I represents the current, and R represents the resistance), the input voltage directly results in a current for the motor 31, independent of a voltage prevailing across the motor 31.

When the current by means of which the motor 31 is operated is kept at a constant value, the output torque of the motor 31 is kept at a constant value, and so is the tension prevailing in the bandage as it is unwound from the roll 10, regardless of other factors including the speed at which the bandage roll 10 is moved in the ring 4. The user can simply use the knob 51 of the potentiometer 50 for setting a desired value of the tension, as the setting of the potentiometer 50 determines the input voltage for the drive circuit 40 and the current at which the electromotor 31 is operated, resulting in a certain output torque of the motor 31 and an associated force acting on the bandage. The controller 41 can be adapted to set a certain predetermined speed for the rotation of the bandage roll 10 and the assembly 30 by means of which the bandage roll 10 is supported and driven under all circumstances, wherein the speed can be chosen such as to be convenient to a user of the bandage applying device 1, i.e. not too fast in order to avoid feelings of discomfort.

If different areas of a limb 2, 3 which is to be enwrapped in a bandage require different tension settings, a user can simply realize the correct values by putting the knob 51 of the potentiometer 50 in appropriate positions during various stages of a bandage applying process. Naturally, the bandage applying device 1 may be equipped with means for allowing a user of the device 1 to stop a bandage applying process and (re)start a bandage applying process at any given time.

It follows from the foregoing that in the bandage applying device 1, it is possible to have accurate control of the tension in a bandage to be applied to a limb 2, 3. This means that treatments of the limb 2, 3 can be most effective, wherein risks that bandages are too loose or too tight are minimized.

It will be clear to a person skilled in the art that the scope of the present invention is not limited to the examples discussed in the foregoing, but that several amendments and modifications thereof are possible without deviating from the scope of the present invention as defined in the attached claims. While the present invention has been illustrated and described in detail in the figures and the description, such illustration and description are to be considered illustrative or exemplary only, and not restrictive. The present invention is not limited to the disclosed embodiments.

Variations to the disclosed embodiments can be understood and effected by a person skilled in the art in practicing the claimed invention, from a study of the figures, the description and the attached claims. In the claims, the word "comprising" does not exclude other steps or elements, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope of the present invention.

For sake of completeness, it is noted that the interface 50 of the bandage applying device 1 does not necessarily need to comprise a potentiometer 50 and an associated knob 51 by means of a setting of the potentiometer 50 can be adjusted. The combination of the potentiometer 50 and the knob 51 is just an example of many possibilities existing within the framework of the present invention, including possibilities of having an interface 50 which is suitable for reading information from a source such as a digital card or the Internet, wherein it is not necessary for a user of the bandage applying device 1 to set input values himself/herself, so that the degree of convenience for a user is increased and mistakes can be avoided. Also, for example, the particulars of the circuit 40 for driving the motor 31 are only provided as an illustration of a practical possibility of realizing accurate control of pressure to be exerted on a limb 2, 3 by a bandage which is applied around that limb 2, 3.

The present invention can be summarized as follows. A device 1 is provided for automatically performing a process of applying a bandage to a limb 2, 3 of a human being or an animal. To that end, the device 1 has a ring 4 which is movably arranged on a guide 5, and which can also be tiltable with respect to the guide 5. The ring 4 can comprise a fixedly arranged basic ring and a rotatable ring portion, wherein a support 34 for supporting a bandage roll 10 is located at the rotatable ring portion. It is also possible that the support 34 itself is arranged such as to be displaceable in the ring 4, wherein an element which is connected to the support 34 at the one hand, and which is movable with respect to the ring 4 on the other hand can be used.

A person can use the device 1 by himself/herself by putting the limb 2, 3 to be enwrapped in the correct position with respect to the device 1 -wherein the limb 2, 3 can be supported by means of a component 7 of the device 1- and activating the device 1. The device 1 then takes care of applying the bandage from the position of the bandage roll 10 as present on the support 34 around the limb 2, 3, wherein the support 34 and the bandage roll 10 as supported by the support 34 rotates around the limb 2, 3, and is gradually displaced along the limb 2, 3. Such a displacement of the support 34 can take place in a direction which substantially equals a direction in which an imaginary axis of the rotary movement of the support 34 around the limb 2, 3 extends. The device 1 is also adapted to guarantee that the pressure in the bandage is realized in an accurate manner, particularly by setting the output torque of a motor 31 which is used for driving the support 34 of the bandage roll 10.

## Claims

1. Device (1) for applying a bandage around a limb (2, 3), comprising:
- a ring (4) which is adapted to surround the limb (2, 3);
- a guide (5) for guiding the ring (4), wherein the ring (4) is movably connected to the guide (5);
- a rotatable support (34) for supporting a bandage roll (10), which support (34) is located in the ring (4);
- a motor (31) for driving the support (34);
- a drive circuit (40) for driving the motor (31);
- an interface (50, 51) for receiving input information representing a desired tension of the bandage during a bandage applying process; and
- a controller (41) for controlling operation of the motor (31) during a bandage applying process through the drive circuit (40), which is connected to the interface (50, 51) for receiving the input information from the interface (50, 51), which is configured to determine a value representing an output torque of the motor (31) on the basis of the input information, and which is connected to the drive circuit (40) for setting the value in the drive circuit (40).

2. Device (1) according to claim 1, wherein the motor (31) is an electromotor, and wherein the controller (41) is configured to determine a value representing a supply of current to the motor (31) on the basis of the input information, and to control the drive circuit (40) for realizing the value.

3. Device (1) according to claim 2, wherein the controller (41) is configured to determine a voltage to be set in the drive circuit (40).

4. Device (1) according to claim 3, wherein the drive circuit (40) comprises a metal-oxide-semiconductor field-effect transistor (MOSFET) (43), an operational amplifier (42) and a resistor (44), wherein the operational amplifier (42) is connected to the controller (41) for receiving an incoming voltage set by the controller (41), and wherein the operational amplifier (42) is arranged for letting a voltage across the resistor (44) be the same as the incoming voltage and thereby determining a value of a current in the drive circuit (40) and determining an opened/closed state of the MOSFET (43).

5. Device (1) according to any of claims 1-4, wherein the interface comprises a potentiometer (50).

6. Device (1) according to claims 1-5, wherein the interface is adapted to communicate with a computer through the Internet.

7. Device (1) according to claim 1-6, wherein the interface is adapted to read information from a digital card.

8. Device (1) according to any of claims 1-7, further comprising a memory for storing information regarding the value representing the output torque of the motor (31) as set in the drive circuit (40).

9. Device (1) according to claim 8, further comprising a transmitter for transmitting information from the memory to a location outside of the device.

10. Device (1) according to claim 9, wherein the transmitter is adapted to transmit information in a wireless fashion.

11. Device (1) according to any of claims 1-10, further comprising a scanner for reading information on a bandage roll (10) representing the type of the bandage roll (10), wherein the scanner is connected to the controller (41).

12. Device (1) according to any of claims 1-11, wherein the ring (4) is tiltable with respect to the guide (5).

13. Device (1) according to any of claims 1-12, wherein the ring (4) has a fixedly arranged basic ring and a rotatable ring portion, and wherein the support (34) for supporting a bandage roll (10) is located at the rotatable ring portion.

14. Device (1) according to any of claims 1-12, wherein the support (34) for supporting a bandage roll (10) is displaceable in the ring (4) by means of an element which is connected to the support (34) on the one hand and which is movable with respect to the ring (4) on the other hand.
